Europäisches Patentamt

European Patent Office

Office européen des brevets

(19)

(11) Publication number: **0 260 599**

**A2**

(12) # EUROPEAN PATENT APPLICATION

(21) Application number: **87113240.3**

(22) Date of filing: **10.09.87**

(51) Int. Cl.⁴: **G01N 33/569** , G01N 33/567 , G01N 33/52

(30) Priority: **12.09.86 DE 3631016**
**30.04.87 DE 3714445**
**19.05.87 DE 3716724**

(43) Date of publication of application:
**23.03.88 Bulletin 88/12**

(84) Designated Contracting States:
**AT BE CH DE ES FR GB IT LI LU NL SE**

(71) Applicant: **DIAGEN Institut für molekularbiologische Diagnostik GmbH**
**Niederheider Strasse 3**
**D-4000 Düsseldorf 13(DE)**

Applicant: **Georg -Speyer-Haus**
**Paul-Ehrlich-Strasse 42-44**
**D-6000 Frankfurt 70(DE)**

(72) Inventor: **Immelmann, Andreas, Dr.**
**Trillser Graben 5**
**D-4006 Erkrath 2(DE)**
Inventor: **Henco, Karsten, Dr.**
**Schlickumer Weg 23**
**D-4006 Erkrath 2(DE)**
Inventor: **von Briesen, Hagen**
**Ringstrasse 31**
**D-6242 Kronberg(DE)**
Inventor: **Aldag, Ulrich, Dr.**
**Schlickumer Weg 13**
**D-4006 Erkrath 2(DE)**
Inventor: **Becker, Walter B., Prof. Dr.**
**10 Reform Road**
**Rondebosch 7700(ZA)**
Inventor: **Rübsamen-Waigmann, Helga, Dr.**
**Königsteiner Strasse 113**
**D-6232 Bad Soden/Taunus(DE)**

(74) Representative: **Werner, Hans-Karsten, Dr. et al**
**Deichmannhaus am Hauptbahnhof**
**D-5000 Köln 1(DE)**

(54) **Method and agent for detecting antibodies against human and animal retroviruses, especially T-lymphotropic viruses.**

(57) Described are a method and means for the detection of antibodies against human and animal retroviruses, and more specifically T-lymphotropic viruses, by allowing a sample to be investigated to react with a human or animal cell line infected with said viruses and subsequently labelling by means of a second antibody directed against human anti- bodies bearing a marker. A cell line is used only part of the cells of which after the infection expresses viral antigens. The negatively-reacting cells are used as internal standard.

# METHOD AND AGENT FOR DETECTING ANTIBODIES AGAINST HUMAN AND ANIMAL RETROVIRUSES, ESPECIALLY T-LYMPHOTROPIC VIRUSES

The present invention relates to a method for the detection of antibodies against human and animal retroviruses, and especially T-lymphotropic viruses, and to an agent for carrying out the method according to the invention. In this method, a sample to be investigated is allowed to react with a human or animal cell line infected with human or animal retroviruses, and more specifically T-lymphotropic viruses. Subsequent labelling of the viral antigens is effected by means of a second antibody directed against human antibodies or antibody fragments bearing a marker.

T-lymphotropic viruses having predominantly a T-cell tropism are pathogens of serious diseases, mostly those of the immune system. In addition, they are also capable of infecting other cell types and furthermore, due to the inherent property of high variability, of modifying and extending their tropism. As the pathogen of the Acquired Immune Deficiency Syndrome (AIDS) there has been identified the Human Immunodeficiency Virus (HIV) belonging to the group comprising retroviruses. This type of viruses infects the T4 helper cells in a patient which are an important subgroup of the T-lymphocytes. AIDS is a disease which is epidemically spreading in nearly all continents.

In addition to the HIV infection, which is infectious by contacts of various infectious body liquids with blood, there appears to occur another worldwide virus epidemic which has already become very common in Africa and the Mediterranean countries.

This virus epidemic is due to an infection by HTLV-I viruses, which may induce a form of T-cell leukemia in adults. Characteristic for this infection is its propagation through blood contacts which corresponds to that of the AIDS virus. Frequently a co-infection by both types of viruses is observed. The incubation period of HTLV-I virus-induced leukemias is between 5 and 30 years. Thus, the epidemiologic significance of this infection is much underestimated.

In the beginning AIDS appeared to be restricted to certain high-risk groups. The abundance of immune deficiency within these high-risk groups already in a very early stage of its general occurence favored the idea that the disease was spread by an infectious factor. Thus, the identification and characterization of the HIV virus as the infectious factor was successfully accomplished.

In the course of the intensive investigation of the disease by Gallo et al., in the publications of the U.S. Department of Commerce, Springfield, National Technical Information Service (NIH) No. SN6-602,945 and No. SN6-602,946 described a method for the in vitro augmentation of AIDS viruses and a method for determining anti-AIDS antibodies in the serum of AIDS patients or of patients suffering from lymphadenopathy. Lymphadenopathy is a disease which generally precedes AIDS.

A neoplastic aneuploid T-cell line isolated from an adult patient suffering from lymphatic leukemia was infected with HIV viruses. The cell line was deposited as H9/HTLV-III$_B$ with the American Type Culture Collection (ATCC, CRL 8543). This permissive cell line was used for the production and isolation of HIV viruses.

Furthermore, by means of the transformed cells which expressed surface antigens specific to HIV viruses, anti-AIDS antibodies could be detected in the serum of patients or test persons.

In SN6-602,945 there has been described an analytical method wherein first virus-specific antigens recovered from cells infected with HIV are isolated and allowed to react with the serum to be investigated. Anti-HIV antibodies, if present in the serum of a test person, are detected by means of radioimmunoassay or the Western blot method. In SN6-602,945 there was also described a determination of anti-HIV antibodies in the serum of a test person by means of ELISA (Enzyme-Linked Immunosorbent Assay) and purified HIV viruses. Moreover, a method was disclosed wherein HIV-virus-infected T-cells are directly allowed to react with the serum of the test person and the antibodies which may be adsorbed on the T-cell surface are detected by means of fluorescent-labelled secondary antibodies. So far ELISA has been in common use as a screening test. The other methods are sometimes very expensive and require technically skilled staff.

The permissive cell line H9 is distinguished by a high virus-specific antigen expression. Almost each cell is infected and is capable of reacting with anti-HIV antibodies if present in the serum. However, the latter property of the H9 line may cause possible false interpretations. If HIV-specific antigen is offered on the surface of each cell and at the same time the anti-HIV antibody titer in the serum of the test person is low, the antibodies will be distributed on too many H9 cells (i.e. diluted), so that they cannot be detected any more with sufficient accuracy by labelling with secondary antibodies.

A signal indicating the presence of anti-HIV antibodies may be lost in the weakly fluorescent background.

As a further disadvantage of the intense antigen expression of the H9 cell line, another phenomenon comes into appearance.

For example, if the serum of the test person contains auto-antibodies against membrane or cell structures such as anti-HLA (Human Leucocyte Antigen) antibody or antimitochondrial antibodies, occurring with symptoms of rheumatic diseases, the surfaces of the H9 cells are loaded with these antibodies. Thereupon, the secondary antibody labels the immunocomplexes adsorbed to the cell and simulates an HV-positive reaction. Therefore, all investigations carried out according to this prior art must be controlled by comparison with non-infected H9 cells. These costly and time-consuming steps render the known methods unattractive for screening programs in tests for T-lymphotropic viruses in body liquids. A severe disadvantage of the described tests consists in the requirement of a high pre-dilution of the serum samples in order to suppress nonspecific reactions. By this pre-treatment a patient's serum having a low antibody titer may be diluted below the limit of detection.

Furthermore, it is desirable to determine in one and the same operation whether a serum sample to be investigated contains anti-HTLV-I and/or anti-HIV antibodies. This parallel determination cannot be carried out with H9 cells as used so far.

A crucial disadvantage of the methods so far known is that highly infectious substances must be handled in the preparations of the reagents and materials for the detection of the antibodies.

The infectious hazard from a virus-infected cell culture is a multiple of that of a serum originating from a patient infected with HIV virus, where the virus titer of said serum is extremely low.

The capability of the viruses to vary their antigenic structures spontaneously or upon induction raises a further serious problem. Thus, it is possible that variants of a kind of pathogenic virus which, though they do not react or give only a weak immunological cross-reaction with the known and characterized virus isolates, cause the same or similar symptoms to occur as those caused by the original virus, but are not detected by the established detection methods. Thus, for example, human and animal retroviruses, and more specifically T-lymphotropic viruses, are known which causatively correlate to diseases like AIDS, ARC (Aids-related Complex) or lymphadenopathy, namely the variants LAV-2 and HTLV-IV of the HIV virus. Antibodies against these variants show only weak immunological reactions or do not show any immunological reactions at all with the known and characterized virus isolates of the HIV virus.

It is the object of the present invention to provide an efficient method of diagnosis and an agent for the detection of antibodies against human and animal retroviruses, and more specifically T-lymphotropic viruses in samples to be investigated, whereby crucial advantages may be achieved over the known methods, such as, for example,

1. the detection of low antibody concentrations in a test serum using undiluted or weakly diluted samples;

2. the detection of antibodies against viral structures from whole blood;

3. the detection of low antibody concentrations by use as internal standard of cells showing no viral antigens in measurable amounts;

4. concentrating low antibody concentrations on a few infected cells;

5. the suppression as extensive as possible of immune reactions with intracellular proteins and cell structures;

6. the exclusion of a false positive signal due to HLA-antibodies present in the sample or auto-antibodies against external membrane structures;

7. the possibility of a simultaneous determination of antibodies against HTLV-I viruses and HIV viruses in one sample to be investigated;

8. the detection of those human and animal retroviruses, more specifically of T-lymphotropic viruses, which are variants of the known HIV viruses, which do not cross-react or cross-react only weakly immunologically with known and characterized virus isolates such as HIV, while, however, they causatively correlate with the diseases AIDS, ARC (Aids related complex) or lymphadenopathy, such as, e.g., the variants LAV-2 or HTLV-IV;

9. the safe detection of the above-mentioned antibodies at an early time, at which ELISA methods fail to provide reliable results;

10. the exclusion of falsely positive Western Blot results;

11. the period of time required for carrying out the test of from 15 to 20 minutes;

12. the usability of samples of most varied origin;

13. the handling of non-infectious material;

14. the high standardization and perfection of the investigation procedures;

15. the simple and safe detection of antibodies against retroviruses from dried traces of blood or blood samples; and

16. the possibility at beginning seroconversion sensitively to detect antibodies against viral nucleus antigens which have been more strongly preserved in various variants and, thus, to be able to detect HIV-I and HIV-II (LAV II) with one virus isolate.

The object of the invention is attained by a method for the detection of antibodies against human and animal retroviruses, and especially T-lymphotropic viruses, by allowing a sample to be investigated to react with a human or animal cell line infected with human or animal retroviruses, and especially T-lymphotropic viruses, and subsequently labelling by means of a second antibody directed against human antibodies or antibody fragments bearing a marker. The method is characterized in that a cell line is used, only part of the cells of which after infection with the human and animal retroviruses, detectably expresses viral antigens and wherein the negatively-reacting cells are used as internal standard.

The method according to the invention in a simple manner enables the diagnostic detection to be accomplished of antibodies against human and animal retroviruses, and especially T-lymphotropic viruses, which due to the unusually high variance of the human and animal retroviruses, and especially T-lymphotropic viruses, such as HIV, do not show any or do only show weak immunolgical reactions with the known and characterized virus antigens. Thus, for example, only virus isolates from blood cells of patients infected with so far unknown human and animal retroviruses, and especially T-lymphotropic viruses, and who show symptoms of AIDS, ARC (Aids related complex) or lymphadenopathy should be used for the infection of the cell lines usable according to the invention. Antibodies against human and animal retroviruses, and more specifically T-lymphotropic viruses such as LAV-2 viruses and HTLV-IV viruses, may be diagnosed.

As the cell lines according to the invention there are employed, for example, HUT 78 (ATCC, TIB I6I), HUT I02 (ATCC, TIB I62) and I9I TJ (ECACC 8609050I). The virus-infected target cells of the type of HUT 78 express viral antigens only at a low percentage (about I to I0%). As could be shown through in situ hybridization, this cell fraction, however, produces more than one thousand times the amount of virus as compared to the remaining 90 to 99% of cells. After labelling the HIV-antigen/anti-HIV-antibody complexes with labelled antibodies against human immunoglobulin, a positive signal can be observed with high sensitivity in comparison with the internal standard of non-antigen-bearing cells. Highly antibody-positive sera can be diluted by the factor of from I:I to I:I0,000 and continue to show an unambiguously identifiable positive reaction. Undiluted sera, whole blood and dried whole blood can also be investigated in this system for antibodies.

The marker of the secondary antibody against the antigen-antibody complexes formed consists of a moiety which either absorbs electromagnetic waves, is fluorescent, chemiluminescent, bioluminescent, radioactive, or is labelled with an enzyme. Thus, known diagnostic methods of clinical chemistry are applicable as suitable detection methods, such as, e.g., RIA (radioimmunoassay), ELISA, EIA, cytofluorimetry, immunofluorescence microscopy and immunofluorescence spectroscopy, as well as methods related thereto, such as cellular RIA and cellular ELISA.

One embodiment of the method of the invention is the simultaneous detection of antibodies against HTLV-I viruses and HIV viruses in one sample. For this purpose, the method of the invention is carried out, for example, by using the cell line HUT I02 (ATCC, TIB I62). This cell line transformed with HTLV-I viruses, which constitutively expresses HTLV-I virus antigens in an amount of more than 50%, has been isolated from a leukemic patient.

It is also possible to use human T-lymphocytes having been transformed with the monkey virus STLV-I (Simian-T-lymphotropic-leukemia virus) in the simultaneous test. This cell line has been deposited at ECACC under the accession number 8609050I. The cells transformed with STLV-I viruses constitutively in an amount of more than 50% express STLV-I antigens exhibiting high homology to the HTLV-I antigens. It has been shown that anti-HTLV-I antiserum cross-reacts with the STLV-I antigens. The simultaneous determination of anti-HTLV-I antibodies besides anti-HIV antibodies in the same sample to be analyzed is simply effected by taking an aliquot of the cells transformed with HTLV-I or STLV-I viruses which constitutively express HTLV-I or STLV-I antigens and is additionally infected in vitro with HIV viruses. The serum of the test person to be investigated is now tested in parallel against HIV-infected and non-infected cells. As the test method there may be adopted a known diagnostic method of clinical chemistry such as, e.g., RIA, ELISA, EIA, cytofluorimetry, immunofluorescence microscopy and immunofluorescence spectroscopy, as well as methods related thereto such as cellular RIA and cellular ELISA. If a positive signal is observed only from the cells having been infected in vitro with HIV, it is to be presumed that in the sample to be investigated only anti-HIV antibodies are contained However, if a positive signal is obtained from the infected as well as the non-infected cells, then this may have been caused by

a) anti-HTLV-I antibodies or

b) anti-HLA antibodies or auto-antibodies against membrane or cell structures such as mitochondrial proteins.

A discrimination of these two possibilities is effected in a confirmation test wherein the serum of the test person is tested against HTLV-I-transformed cells without a HIV co-infection and against non-transformed peripheral T-lymphocytes. This embodiment of the method of the invention also makes it possible to detect potentially infectious serum samples containing HTLV-I.

The preferred embodiment the method of the invention offers the further advantage of handling infected cells which, however, are no longer infectious. Nevertheless, the antigenic structures are presented unchanged within the sense that the reaction of the antibodies against human and animal retroviruses, and more specifically T-lymphotropic viruses, takes place with the corresponding antigenic structures on the cell.

The methods used so far for the fixation of cells for immunofluorescence, which were also employed for the detection of anti-HIV antibodies, e.g. acetone fixation of native cells on glass or reductive treatment of the cells with detergents such as SDS (sodium dodecylsulfate) in Western Blots, destroy the superordinate structures of the antigens.

Thus, antibodies requiring intact conformations of the antigen for binding remain undetected by the method using antigen-antibody complexes.

From electron microscopy there have been known methods for an in situ fixation which causes the complex molecular structures of biological material to be mostly retained with respect to their spatial arrangement and structures. In immunological research in situ fixation has already been employed in the mixed lymphocyte reaction using fixed stimulator cells. Surprisingly it has been shown that an in situ fixation of the cells infected with human and animal retroviruses, and more specifically T-lymphotropic viruses, does not decrease the sensitivity of the detection of antibodies against human and animal retroviruses, and more specifically T-lymphotropic viruses, such as HTLV-I, STLV-I as well as HIV viruses. Aldehydes, and more specifically paraformaldehyde, formaldehyde and/or glutaraldehyde, have proven to be suitable fixing agents for the in situ fixation in the method of the invention, as long as said aldehydes will not spectroscopically interfere with the optical detection methods.

The surprising finding of an unreduced sensitivity of the detection of antibodies against human and animal retroviruses, and especially T-lymphotropic viruses, upon the use of in situ-fixed cells may possibly be interpreted on the basis that the newly formed virions and fragments of virions are vesicularly concentrated in the outer cell membrane and, thus, remain capable of reacting with bulky antibodies in spite of a plastification of the interior of the cell. This will be especially applicable to all viruses of the HIV type, lentiviruses, spumaviruses and HTLV-I viruses.

Cross-reactions with internal antigens, e.g. with mitochondrial antigens, are efficiently suppressed.

Another surprising finding in the analysis of a sera of an early seroconverter which, upon Western Blot analysis, only shows antibodies against HIV nucleus antigens, is that those antibodies are also sensitively detected after fixation although they must be supposed to be present within the virion structure.

On the use of these cells in fluorescence spectroscopical equipment, such as FACS and fluorescence spectrometers, even a sensitive and automatizable signal quantification becomes possible which allows concentrations of antibodies to be determined over a wide range thus far not possible with the use of ELISA methods.

Thereby it becomes possible to produce test kits extensively avoiding operations using highly pathogenic solutions or materials. If cells having been thus fixed are employed, it is surprisingly still possible to carry out the immunological reactions not only on a solid phase, but also in solution using non-infectious reagents in the form of free but plasticized cells. However, if desired the cells may also be subsequently immobilized on surfaces such as, for example, glass or may adhere to such surfaces.

Such a test kit advantageously contains fixed cells of a cell line infected with human and animal retroviruses, and especially T-lymphotropic viruses, wherein the cell line expresses viral antigens only in part detectably, and furthermore fixed cells of a cell line transformed by human and animal retroviruses, and more specifically T-lymphotropic viruses, whereby the cell line constitutively expresses viral antigens in an amount of more than 50%, and secondary antibodies against human antibodies, which secondary antibodies bear a marker.

The cell line infected with human and animal retroviruses, and especially T-lymphotropic viruses, may be, e.g., of the kind of HUT 78, as well as a cell line transformed by human and animal retroviruses, and more specifically T-lymphotropic viruses, of the kind HUT I02 and/or I9I TJ. Cells transformed by human and animal retroviruses, and more specifically T-lymphotropic viruses, in particular are those of the cell lines HUT I02 and I9I TJ.

Furthermore, by the means of the invention there may also be adapted also established methods of qualitative and quantitative diagnostics such as RIA, ELISA, EIA, FACS, immunofluorescence microscopy and immunofluorescence spectroscopy, as well as methods related thereto such as cellular RA and cellular ELISA. More specifically, if immunofluorescence microscopy is employed, the properties of the cell line HUT 78, usable according to the invention, are of great advantage. The HIV antigens are homogeneously distributed over the plasma membrane of the infected cells. Therefrom ensue three characteristic criteria for assessment in the evaluation of the immunofluorescence test:

I. Only from I to l0% of all cells are fluorescent;

2. fluorescence occurs bound to the membrane and appears as a ring fringing the cell;

3. the fluorescence is not subject to capping phenomena, but occurs in a homogeneous distribution.

The virus-infected HUT 78 cell line expresses HIV-specific antigens in only I to l0% of the cells.

Thus, for anti-HIV antibodies possibly present in the serum an excess of the corresponding binding partner is available only on a few cells. For example, a fluorescence signal is not homogeneously distributed throughout the entire cell population, but it is concentrated only on cells expressing antigens. The effect comes into appearance particularly clearly by the labelling of the polynuclear syncytia. The local accumulation of the viral antigens results in a considerable increase in sensitivity of the investigation method.

Due to the high sensitivity of the HIV immunofluorescence test the assessment is possible even for a test person's serum which did not give certain results in other confirmation tests. Thus, among II9 samples from blood banks there were found three serum samples which had been rated as antibody-positive, which, however, obtained an unambigously negative rating by the method of the invention. Upon inquiry, it became evident that said serum samples had originated from donors who did not belong to any high risk group, who did not show any clinically striking features, and for whom an infection with HIV viruses was to be considered as unlikely. Thus, in the first screening test these samples had given positive results for antibodies; subsequent tests for confirmation showed contradictory results which, however, finally resulted in an obviously false classification of said serum samples as being "antibody-positive". The method of the invention shows a reliable performance also with samples taken from body liquids processed in the most varied manners (dry blood traces, mortified tissue, sputum, saliva, sperm etc.). Since the method of the invention also allows a safe detection of antibodies against HIV viruses in dry blood, a blood sample may be collected in a doctor's office and shipped to a diagnosis center.

Thereby, an anonymous and confidential treatment of a test person is assured. Moreover, serial investigations may be carried out in underdeveloped countries of the Third World. Since the samples are stable for a period of several weeks (room temperature) they may be collected and mailed to central places for diagnosis. It is convenient to employ the following means for mailing the samples:

I. A plastic film with holes (3 mm in diameter) in the format of a slide mask and small filter disks, for example 3MM Whatman filters (5 mm diameter) glued to the edges of the holes (see Figures la and lb) or

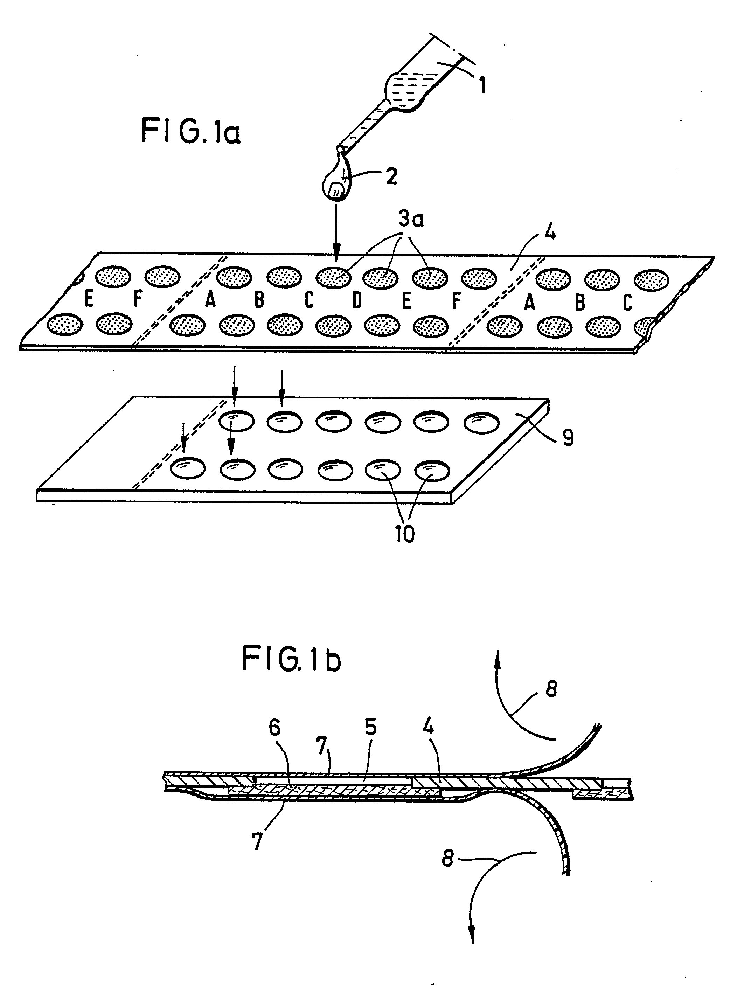

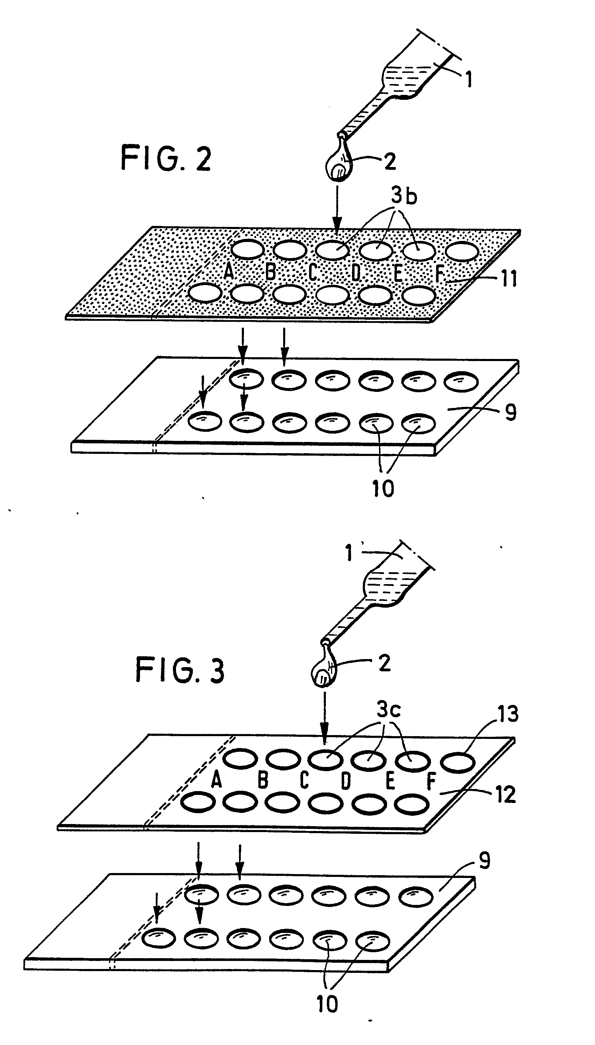

2. filters made of stiff paper, for example 3MM filters (Whatman) with slide mask formatting wherein either the total mask has been plasticized or rendered hydrophobic or the filter disk label has been applied by using a hydrophobic printing material capable of penetrating the filter (see Figures 2 and 3).

However, all means are suitable which prevent serum constituents or solutions of the elution buffers from inadvertently causing any interfering contaminations.

The Figures show devices for receiving a blood sample which is storable as a dry blood sample and is suitable to be mailed or shipped without being cooled. A blood sample is contained in a capillary I. From the capillary a small amount of blood 2 is dropped into fields 3a, 3b, 3c provided therefor.

The Figures la and lb show a continuous plastic film 4 perforated in a transverse direction, which film comprises recesses 5 adhesion-sealed with an absorptive material, and preferably with small disks 6 made of filter paper (Fig. lb), said recesses 5 together forming the field 3a. The sizes of the recesses 5 are adapted to correspond to the sizes of the respective slide masks used in the analysis and are preferred to have a diameter of 3 mm. The small filter disks then preferably have diameters of about 5 mm. The upper and lower sides of this device, if desired, may each be protected by a removable cover foil 7 which, for example, is strippable the direction as indicated by the arrows 8 in Fig. lb. The blood sample is dropped onto the corresponding areas 3a and then allowed to dry. In the analysis carried out later, the device for carrying out the method of the invention is directly placed onto a slide 9 so that the depressions l0 in the slide are brought into a coaxial relation, and preferably congruence, with the respective areas 3a. Of course, the strippable cover film 7, if present, must have been previously removed. Then the small filter disk is eluted by means of a suitable buffer.

In Figure 2 the layer bearing the field 3b provided to receive the sample is preferably a continuous paper strip II with transverse perforation; said paper strip has been impregnated with a hydrophobic material, except for the areas 3b which preferably are of circular shape, the hydrophobic material penetrating the paper and covering both surfaces.

The hydrophobic material is applied, for example, by means of a printing process. The paper strip II may consist, for example, of paper, Whatman grade 3MM, and may also be protected by plastic cover foils.

Figure 3 schematically shows a further preferred embodiment of the device for practicing the method of the invention.

Herein the layer bearing the field 3c provided to receive the blood sample is preferably a continuous paper strip I2 with transverse perforations at definite sections; the areas 3c, preferably of round shape, are defined by a preferably circular line I3 consisting of hydrophobic material. The hydrophobic material must be superficially applied onto the paper strip I2 as well as penetrate the paper strip at the marked locations. The hydrophobic material preferably is a hydrophobic print material. The sizes of the areas 3b and 3c in the corresponding embodiments are also adapted to be made congruent with the depressions I0 of the slides 9 as used in each case.

A further preferred embodiment of the device for carrying out the method of the invention consists of a laminate comprising

　　　a) an upper hydrophobic foil including recesses;

　　　b) a lower hydrophobic film including recesses coaxial to the recesses in the upper hydrophobic foil;

　　　c) an absorptive material positioned therebetween which on all sides projects beyond the edges of the recesses of the foil; and

　　　d) optionally an upper and/or lower removable cover foil applied over the upper and/or lower hydrophobic foil.

A further device for carrying out the method of invention consists of a strip-shaped film onto which pieces of an absorptive material have been applied in spaced relations, or a rod-like arrangement at the end of which the absorptive material has been located. The width of the strip-shaped film provided with the pieces of an absorptive material is determined by the number of tests desired.

The devices for carrying out the method of the invention may be available either as pieces or in form long strips capable of being wound up.

The present invention is further illustrated by means of the following examples.

The human T-cell line I9I TJ has been deposited at the European Collection of Animal Cell Cultures, Porton Down, Salisbury, Wiltshire SP 4 OJG in England under the number 86090501 on September 05, I986. The human cell line HUT 78 has been deposited under the number ATCC TIB I6I, and the human cell line HUT I02 has been deposited under the number ATCC TIB I62 at the American Type Culture Collection, I2301 Parklawn Drive, Rockville, Maryland 20852, U.S.A. (ATCC Catalogue, 5th Edition I985, p. 239).

EXAMPLE I

The permanent line I9I TJ (ECACC, 8609050I) was established by co-cultivating human umbilical cord cells with peripheral lymphocytes of a monkey (Cercopithecus aethiops pygerythrus), who had been infected with the STLV-I virus (Simian T-cell Leukemia Virus I).

The cultivation of these cells is effected in RPMI-I640 medium (Gibco, Paisley, Scotland) with the following additives: 0.2% of $NaHCO_3$; 20% of fetal calf serum, 2% of L-glutamine; I00 I.U./ml of penicillin as well as 60 micrograms/ml of streptomycin. The cells are incubated in an atmosphere saturated with water vapor and containing 5% by volume of $CO_2$ at 37 °C.

An infection of these cells with the HIV virus is effected by co-cultivating the cell line I9I TJ with a culture of peripheral lymphocytes of an AIDS patient. Peripheral lymphocytes of the patient P 34, who had been infected by the HIV virus, were isolated according to the Ficoll-Hypaque method and incubated in culture medium RPMI-I640 additionally containing 20% of T-cell growth factor and I00 micrograms/ml of phytohaemagglutinin for some days. Cells of the line I9I TJ (ECACC, 8609050I) and the activated infected lymphocytes were mixed in a ratio of I:5 and further cultivated.

EXAMPLE 2

The cell lines HUT 78 (ATCC, TIB I6I) and HUT I02 (ATCC, TIB I62) were isolated from patients suffering from a T-cell leukemia. The cultivation of these cells is effected in RPMI-I640 medium with the following additives: 0.2% of $NaHCO_3$; 20% of fetal calf serum; 2% of L-glutamine; I00 I.U./ml of penicillin and 60 micrograms/ml of streptomycin. The cells were incubated in an atmosphere saturated with water vapor and containing 5% by volume of $CO_2$ at 37 °C.

An infection of these cells with HIV virus is effected by co-cultivating the cell lines HUT 78 (ATCC, TB l6l) or HUT l02 (ATCC, TIB l62) with a culture of peripheral lymphocytes of an AIDS patient. Peripheral lymphocytes of the patient P 34 infected with the HIV virus were isolated according to the Ficoll-Hypaque method and incubated in culture medium RPMI-l640 additionally containing 20% of T-cell growth factor and l00 micrograms/ml of phytohaemagglutinin for some days. Cells of the lines HUT 78 (ATCC, TIB l6l) or HUT l02 (ATCC, TIB l62) and the activated infected lymphocytes were mixed in a ratio of l:5 and further cultivated.

## EXAMPLE 3

The fixation of HIV-infected human lymphocytes or syncytia in suspension with aldehydes is effected as follows:

Human T-lymphocytes, e.g. of the kind of the HUT 78 (ATCC, TIB l6l) cells, and human T-lymphocytes of the kind of the HUT 78 (ATCC, TIB l6l) cells which had been infected with HIV viruses in accordance with the procedure of EXAMPLE 2 are collected by centrifugation (5 minutes at 250 g), washed twice in phosphate-buffered saline (PBS) and adjusted to a cell density of $4 \times l0^7$/ml of in PBS. Aldehydes such as formaldehyde, paraformaldehyde or glutaraldehyde are added to make a concentration of 4%. Then the cells are fixed at ice bath temperature for 30 to 60 minutes and then washed twice in PBS.

## EXAMPLE 4

The fixation of HIV-infected/STLV-I transformed or HIV-infected/HTLV-I transformed human lymphocytes or syncytia, respectively, in suspension with aldehydes is effected as follows:

Human T-lymphocytes, e.g. of the kind of the l9l TJ (ECACC, 8609050l) cells or the HUT l02 (ATCC, TIB l62) cells, and human T-lymphocytes of the kind of the l9l TJ cells and the HUT l02 (ATCC, TIB l62) cells which had been infected with HIV viruses in accordance with the procedures of EXAMPLES l and 2 are collected by centrifugation (5 minutes at 250 g), washed twice in phosphate-buffered saline (PBS) and adjusted to a cell density of $4 \times l0^7$/ml of in PBS.

Aldehydes such as formaldehyde, paraformaldehyde or glutaraldehyde are added to make a concentration of 4%. Then the cells are fixed at ice bath temperature for 30 minutes and then washed twice in PBS.

## EXAMPLE 5

The coating of masked slides with HIV, HIV/STLV-I or HIV/HTLV-I infected, highly infectious cells and the fixation is effected as follows:

Uninfected cells of the kind of HUT 78 (ATCC, TIB l6l), HUT l02 (ATCC, TIB l62) and l9l TJ and cells of the kind of the HUT 78 (ATCC, TIB l6l), HUT l02 (ATCC, TIB l62) and l9l TJ, which had been infected either with HIV (HUT 78) or with HIV and STLV-I (l9l TJ) or with HTLV-I and HIV (HUT l02) in accordance with the procedures of EXAMPLES l and 2 are collected by centrifugation (5 minutes at 250 g), washed twice in phosphate-buffered saline (PBS) and adjusted to a cell density of $2 \times l0^6$/ml of in PBS.

For the immunofluorescence test there are used masked slides with l2 application areas, 5 mm in diameter each. Ten microliters of the cell suspensions of infected and uninfected cells are pipetted per application site onto the slide: The application sites l to 6 are provided with uninfected cells. The application sites 7 to l2 are supplied with infected cells. The cells are dried in air for 30 minutes and then fixed in anhydrous acetone precooled to -80 °C for l0 minutes. Coated slides are stored at -20 °C.

## EXAMPLE 6

The coating of masked slides with non-infectious HIV, HIV/STLV-I or HIV/HTLV-I infected cells having been pre-fixed in situ is effected as follows:

Human T-lymphocytes, e.g. of the kind of the HUT 78 (ATCC TIB l6l) cells, the l9l TJ cells and the HUT l02 (ATCC TIB l62) cells and human T-lymphocytes of the same kind, which had been infected in accordance with the procedures of EXAMPLES l and 2 and had been fixed in situ in accordance with the procedures of EXAMPLES 3 and 4, are adjusted to a cell density of $2 \times l0^6$/ml with PBS. For the immunofluorescence test there are used masked slides with l2 application areas, 5 mm in diameter each. Ten microliters of the cell suspensions of infected and uninfected cells are pipetted per application site onto the slide: The application sites l to 6 are provided with uninfected cells. The application sites 7 to l2 are supplied with infected cells. The cells are dried in air for 30 minutes and then fixed in anhydrous acetone precooled to -80 °C for l0 minutes. Coated slides are stored at -20 °C.

## EXAMPLE 7

The immunofluorescence test for the in vitro determination of anti-HIV-antibodies in serum is carried out as follows:

The slides are prepared in accordance with EXAMPLES 5 or 6. In a first incubation step the anti-HIV-antibodies are bound to virus proteins integrated in the plasma membrane of infected cells of the kind of the HUT 78 cells.

In a second incubation step fluorescent-labelled antibodies directed against human immunoglobulin are bonded to the bound antibodies. The amount of bonded secondary antibody depends on the anti-HIV-antibody content of the serum sample. Non-bonded secondary antibodies are removed by washing. Cell-bound fluorescence is detected by means of a fluorescence microscope.

As reagents there are used:
Phosphate-buffered saline
Bovine serum albumin
Antibody-FITC-conjugate          (Fluorescein isothiocyanate)
Glycerol

Preparation of the solutions:
I Phosphate-buffered saline:
Dissolve 6 g of NaCl, I.8 g of $K_2HPO_4 \times 3H_2O$ and 0.27 g of $KH_2PO_4$ in I liter of twice distilled water.
II Sample buffer:
Dissolve 0.5 g of bovine serum albumin in 50 ml of solution I.
III Antibody-FITC-conjugate:
Mix I part by volume of the anti-IgG-antibody-FITC-conjugate with 40 parts by volume of solution II.
IV Cover solution:
Mix 9 parts by volume of glycerol with I part by volume of solution I.

For the preparation of the sample, I part by volume of serum of the test person is mixed with 9 parts by volume of solution II.

The test is carried out at room temperature, I8 to 25 °C:

-Ten microliters of the diluted serum of the test person are dropped onto one application site containing infected cells. Another I0 microliters are to be pipetted onto the adjacent application site which contains uninfected cells. On each slide two positions are provided for a positive serum and negative serum as controls. The slide is incubated in a moist chamber at 37 °C for 30 minutes.

-Then the slide is washed in 50 ml of solution I for 5 minutes. The washing operation is repeated once. Thereafter the slide is allowed to dry in the air.

-Fifteen microliters of antibody-conjugate (solution III) are pipetted onto the application sites. Then the slide is incubated in a moist chamber at 37 °C for 30 minutes.

- The slide is then washed in 50 ml of solution I for 5 minutes. The washing operation is repeated once. Thereafter the slide is allowed to dry in the air.

-Five microliters of solution IV are pipetted onto the application sites and these are covered with cover slips. The slide is observed in a fluorescence microscope at a total magnification of 200X using the filter combination for fluorescein-isothiocyanate. Positively rated, and, thus, indicative of the presence of anti-HIV-antibodies, are those fuorescence phenomena which occur in an annular shape (ring shape) on the infected cells. If a serum causes a fluorescence coloration to occur on infected as well as uninfected cells, then it has to be presumed that the serum contains cross-reacting antibodies which do not correlate with a HIV infection.

## EXAMPLE 8

Immunofluorescence test for the in vitro determination of anti-HIV-antibodies in undiluted serum.

In accordance with the procedure of EXAMPLE 7 the detection of antibodies against HIV is carried out as follows:

Slides are prepared in accordance with the EXAMPLES 5 or 6. Infected and uninfected cells, respectively, are each reacted with I0 microliters of the undiluted serum to be investigated at 37 °C for 5 minutes. Unbonded serum components are rinsed with PBS (phosphate-buffered sodium chloride solution; solution I).

Then the slide is incubated in PBS, which has been previously warmed to 37 °C, for 5 minutes. After the slide has been dried, the bonded anti-HIV-antibodies are reacted with a fluorescent-labelled anti-human immunoglobulin-antibody at 37 °C for 5 minutes. After the slide has been washed with PBS, the test evaluation is conducted using a fluorescence microscope in the same manner as in EXAMPLE 7.

## EXAMPLE 9

Immunofluorescence test for the in vitro determination of anti-HIV-antibodies in whole blood.

In accordance with the procedure of EXAMPLE 8 the detection of antibodies may be carried out in such a way that 20 microliters of whole blood, and preferably whole blood comprising anticoagulants, are employed in each testing operation.

## EXAMPLE 10

Immunofluorescence test for the in vitro determination of anti-HIV-antibodies within twenty minutes.

In accordance with the procedures of EXAMPLES 8 and 9 the detection of antibodies can be carried out in such a way that by using undiluted serum or whole blood safe results on the presence of anti-HIV-antibody in the sample being investigated are obtained after 20 minutes. Since the use of whole blood steps of sample preparation inherently necessary for other detection systems, such as centrifugation and sample dilution, are dispensable, it is possible to obtain a result already 20 minutes after the collection of the sample.

## EXAMPLE 11

The immunofluorescence test for the simultaneous in vitro determination of anti-HIV-antibodies and anti-HTLV-I-antibodies in serum is carried out as follows:

Test principle of the immunofluorescence test on slides prepared in accordance with EXAMPLES 5 or 6:

In a first incubation step the anti-HIV-antibodies and/or anti-HTLV-I antibodies present in the serum are bound to virus proteins integrated in the plasma membrane of infected cells of the kind of I9I TJ (ECACC, 8609050I) cell or HUT I02 (ATCC TIB I62) cell.

In the second incubation step fluorescent-labelled antibodies directed against human immunoglobulin are bonded to the bound antibodies. The amount of bonded secondary antibody depends on the anti-HIV-antibody content and/or anti-HTLV-I antibody content of the serum sample. Non-bonded secondary antibodies are removed by washing. Cell-bound fluorescence is detected by means of a fluorescence microscope.

The test is conducted as described in EXAMPLE 7.

## EXAMPLE 12

The immunofluorescence test as a suitable means for elucidating questionable results from preceding confirmation investigations (Western-Blot).

Falsely negative Western-Blot results:

Western-Blot results are rated to be anti-HIV-antibody-positive beyond doubt only if in the investigated sample antibodies against at least one species of viral shell proteins (gp 4I, gp II0/I20, gp I60) can be detected. Samples which only contain antibodies against viral core proteins were falsely rated as anti-HIV-antibody negative in the Western-Blot, whereas by means of the immunofluorescence test they could be correctly identified as antibody-positive (for example serum I5869 from the Georg-Speyer-Haus/Frankfurt; antibody only against the virus protein PI8).

Falsely positive Western-Blot results:

If the sample investigated contains antibodies against cell-internal structures, then false positive findings may be simulated in the Western-Blot (New England Journal of Med. 3I4, II8, I986; Saag et al.). By the manner of cell fixation as described in EXAMPLES 3 and 4 the non-specific binding of those antibodies to infected cells, which had been reacted with serum to be investigated in accordance with the EXAMPLES 7 to II is greatly reduced.

## EXAMPLE 13

The immunofluorescence test is capable of identifying one serum or another as being anti-HIV-antibody positive in an earlier stage than conventional ELSA screening tests.

Due to the specific cell-virus system (a few cells bearing antigens among an abundance of cells bearing no antigens) and due to the preservation of the native structure of viral proteins as described in the EXAMPLES 3 and 4, in several cases serum samples which in preceding ELISA screening tests had been found to be below the cut-off point could be rated as unambiguously anti-HIV-antibody positive. It was only in subsequent investigations of these patients that positive results were also obtained in the ELISA tests.

## EXAMPLE 14

The immunofluorescence test is capable of identifying HIV-2 serum samples which were negative in conventional ELISA screening tests based on HIV-I as in fact being anti-HIV-antibody positive.

Due to the virus described in the EXAMPLES I and 2 regarding cell infection and due to the preservation of the native structure and native quantitative antigen pattern of viral proteins by means of the cell fixation as described in the EXAMPLES 3 and 4, in several cases HIV-2 serum samples which in preceding ELISA screening tests had been found to be below the cut-off point could be rated as unambiguously anti-HIV-antibody positive.

EXAMPLE 15

The cellular ELISA for the in vitro determination of anti-HIV and/or anti-HTLV-I antibodies in serum is carried out as follows:

In accordance with the EXAMPLES 7 and II the detection of serum antibodies against HIV and/or HTLV-I is effected in a cellular ELISA, wherein these antibodies may be bound to cells the infection of which has been described in the EXAMPLES I and 2. Cells having thus been infected and uninfected cells of the same kind are fixed on suitable solid carriers according to EXAMPLES 5 or 6 and thereon reacted with the serum samples to be investigated:

In a first incubation step anti-HIV-antibodies present in the serum are bound to virus proteins integrated in the plasma membrane of infected cells of the kind of HUT 78 (ATCC TIB I6I) cells or anti-HIV and/or anti-HTLV-I antibodies to virus proteins integrated in the plasma membrane of infected cells of the kind of the I9I TJ (ECACC, 8609050I) cells or the HUT I02 (ATCC, TIB I62) cells.

In a second incubation step peroxidase-labelled antibodies directed against human immunoglobulin are bonded to the bound antibodies.

The amount of bonded secondary antibody depends on the anti-HIV-antibody content and/or anti-HTLV-antibody content of the serum sample. Non-bonded secondary antibodies are removed by washing. The cell-bound peroxidase oxidizes diaminobenzidine which in the oxidized state precipitates at the site of the bonded antibody. The test is carried out using slides as solid carriers for fixed cells.

As reagents there are used:
Phosphate-buffered saline
Bovine serum albumin
Antibody-peroxidase-conjugate
Diaminobenzidine
Tris-buffer
Glycerol
$H_2O_2$

Preparation of the solutions:

I Phosphate-buffered saline:
Dissolve 6 g of NaCl, I.8 g of $K_2HPO_4 \times 3H_2O$ and 0.27 g of $KH_2PO_4$ in I liter of twice distilled water.
II Sample buffer:
Dissolve 0.5 g of bovine serum albumin in 50 ml of solution I.
III Antibody-peroxidase-conjugate:
Mix I part by volume of the anti-IgG-antibody-peroxidase-conjugate with 40 parts by volume of solution II.
IV Tris-HCl solution:
Dissolve 6 g of Tris in 800 ml of twice distilled water, adjust the pH value to 7.5, fill to make I liter.
V Diaminobenzidine solution:
Dissolve 0.I g of diaminobenzidine in I00 ml of solution IV, adjust the pH value to 5.5.
VI Cover solution:
Mix 9 parts by volume of glycerol with I part by volume of solution I.

For the preparation of the sample I part by volume of serum of the test person is mixed with 9 parts by volume of solution II.

The test is carried out at room temperature, I8 to 25 °C:

-Fifteen microliters of the diluted serum of the test person are to be dropped onto one application site containing infected cells. Another I5 microliters are to be pipetted onto the adjacent application site which contains uninfected cells. On each slide two positions are provided for a positive serum and negative serum as controls. The slide is incubated in a moist chamber at 37 °C for 30 minutes.

-Then the slide is washed in 50 ml of solution I for 5 minutes. The washing operation is to be repeated once. Thereafter the slide is allowed to dry in the air.

-Fifteen microliters of antibody-conjugate (solution III) are to be pipetted onto the application sites. Then the slide is incubated in a moist chamber at 37 °C for 30 minutes.

-The slide is then washed in 50 ml of solution I for 5 minutes. The washing operation is to be repeated once.

-Thirty microliters $H_2O_2$ are added to I00 ml of solution V. The solution is thoroughly mixed, and the slide is incubated in this solution under protection from light for 30 minutes.

-The slide is then washed in 50 ml of solution I for 5 minutes. The washing operation is to be repeated once. Thereafter the slide is allowed to dry in the air.

Five microliters of solution IV are to be pipetted onto the application sites and these are to be covered with cover slips.

The slide is observed in a light microscope at a total magnification of 200X. Positively rated, and, thus, indicative of the presence of anti-HIV-antibodies and/or anti-HTLV-I-antibodies, are dark precipitates which occur in an annular shape on the infected cells. If a serum causes a coloration to occur on infected as well as uninfected cells, it has to be presumed that the serum· contains cross-reacting antibodies which do not correlate with a HIV infection. As an alternative, a phosphatase-labelled secondary antibody and respective substrates may be employed.


## EXAMPLE I6

The ELISA for the in vitro determination of anti-HIV and/or anti-HTLV-I antibodies in serum is carried out as follows:

In accordance with EXAMPLE I5, serum antibodies against HIV and/or HTLV-I can be detected by reacting them in a secondary reaction with phosphatase-labelled antibodies directed against human immunoglobulin. The amount of bonded secondary antibody depends on the anti-HIV-antibody content and/or anti-HTLV-antibody content of the serum sample. Non-bonded secondary antibodies are removed by washing. The cell-bound phosphatase converts a substrate which absorbs visible light. This absorption of light corresponding to the amount of bonded serum antibody is quantified by means of a photometer.

However, for this kind of detection system it is preferred to employ a cell line which expresses viral antigens at a higher frequency such as, e.g., cloned and selected variants of the cell lines I9I TJ (ECACC, 8609050I) and HUT 78 (ATCC, TIB I6I).

The test is carried out using slides as solid carriers for fixed cells.

As reagents there are used:
Phosphate-buffered saline
Bovine serum albumin
Antibody-phosphatase-conjugate
p-Nitrophenyl phosphate
Diethanolamine buffer

Preparation of the solutions:

   I Phosphate-buffered saline:
Dissolve 6 g of NaCl, I.8 g of $K_2HPO_2 \times 3H_2O$ and 0.27 g of $KH_2PO_4$ in I liter of twice distilled water.
   II Sample buffer:
Dissolve 0.5 g of bovine serum albumin in 50 ml of solution I.
   III Antibody-phosphatase-conjugate:
Mix I part by volume of the anti-IgG-antibody-phosphatase-conjugate with 40 parts by volume of solution II.
   IV Diethanolamine buffer:
A IM solution of diethanolamine containing $MgCl_2$ in a concentration of 0.5 mM is adjusted to pH 9.8.
   V p-Nitrophenyl phosphate solution:
Dissolve 0.I g of p-nitrophenyl phosphate in I00 ml of solution IV.

For the preparation of the sample I part by volume of serum of the test person is mixed with 9 parts by volume of solution II.

The test is carried out at room temperature, I8 to 25 °C:

-Fifteen microliters of the diluted serum of the test person are to be dropped onto one application site containing infected cells. Another I5 microliters are to be pipetted onto the adjacent application site which contains uninfected cells. On each slide two positions are provided for a positive serum and negative serum as controls. The slide is incubated in a moist chamber at 37 °C for 30 minutes.

-Then the slide is washed in 50 ml of solution I for 5 minutes. The washing operation is to be repeated once. Thereafter the slide is allowed to dry in the air.

-Fifteen microliters of antibody-conjugate (solution III) are to be pipetted onto the application sites. Then the slide is incubated in a moist chamber at 37 °C for 30 minutes.

-The slide is then washed in 50 ml of solution I for 5 minutes. The washing operation is to be repeated once.

-The slide is allowed to dry. 60 microliters of freshly prepared solution V are pipetted onto each application area, and the slide is incubated in a moist chamber for 30 minutes.

-From each application 50 microliters of the substrate solution are taken and measured in a spectrophotometer at a wavelength of 405 nm.

The amount of reacted substrate and, thus, the light absorption at 405 nm corresponds to the amount of anti-HIV-and/or anti-HTLV-I-antibodies present in the serum. The light absorption caused by a serum to be investigated of a test person is compared to the absorption caused by a negative serum included in the test.

EXAMPLE I7

The cytofluorimetric investigation for the in vitro determination of anti-HIV and/or anti-HTLV-I-antibodies in serum is carried out as follows:

Human T-lymphocytes, e.g. of the kind of the HUT 78 (ATCC, TIB I6I) cells, the HUT I02 (ATCC, TIB I62) cells or the I9I TJ (ECACC, 860905OI) cells and human T-lymphocytes of the same kind, which had been infected in accordance with the procedures of EXAMPLES I and 2 and had been fixed in situ in accordance with the procedures of EXAMPLES 3 and 4, are reacted with the serum to be investigated.

In the first incubation step the anti-HIV-antibodies and/or anti-HTLV-I antibodies present in the serum are bound to virus proteins integrated in the plasma membrane of infected cells.

In the second incubation step fluorescent-labelled antibodies directed against human immunoglobulin are bonded to the bound antibodies. The amount of bonded secondary antibody depends on the anti-HIV-antibody content and/or anti-HTLV-I antibody content of the serum sample. Non-bonded secondary antibodies are removed by washing. Cell-bound fluorescence is detected by means of a fluorescence-activated cell sorter.

The evaluation is effected by counting I0,000 to 20,000 individual cells and calculating the relation of the fluorescence intensity of each individual cell in comparison to the fluorescence intensity of uninfected cells. However, for this kind of detection system it would be preferred to employ a cell line which expresses viral antigens at a higher frequency such as, e.g., cloned and selected variants of the cell lines I9I TJ (ECACC, 860905OI) and HUT 78 (ATCC, TB I6I).

As reagents there are used:
Phosphate-buffered saline
Bovine serum albumin
Antibody-FITC-conjugate
A suspension of fixed cells in a concentration of 4 $\times$ I0$^7$/ml

Preparation of the solutions:

I Phosphate-buffered saline:
Dissolve 6 g of NaCl, I.8 g of K$_2$HPO$_4$ $\times$ 3H$_2$O and 0.27 g of KH$_2$PO$_4$ in I liter of twice distilled water.
II Sample buffer:
Dissolve 0.5 g of bovine serum albumin in 50 ml of solution I.
III Antibody-FITC-conjugate:
Mix I part by volume of the anti-IgG-antibody-FITC-conjugate with 40 parts by volume of solution II.

For the preparation of the sample I part by volume of serum of the test person is mixed with 9 parts by volume of solution II.

The test is carried out at room temperature, I8 to 25 °C:

-Fifty microliters of the diluted serum of the test person are admixed with 25 microliters of a cell suspension of infected and uninfected cells.

Parallel thereto control batches are to be run with positive serum and negative serum. The batches are incubated at 37 °C for 30 minutes.

- The cells are sedimented by centrifugation, washed twice in solution II and re-suspended in 20 microliters each of solution I.

-Fifty microliters of antibody-conjugate are added to each batch; the batches are incubated at 37 °C for another 30 minutes.

-The cells are sedimented by centrifugation, washed twice in solution II, re-suspended in I00 microliters of solution I and analyzed in the cell sorter.

EXAMPLE I8

The fluorescence spektroscopic detection for the in vitro determination of anti-HIV and/or anti-HTLV-I antibodies in serum is carried out as follows:

In accordance with the procedure of EXAMPLE I7 the cell-bound fluorescence may also be detected in a photometer. The cells labelled with a fluorescent secondary antibody are subjected to a fluorescence-spectroscopic investigation, wherein the emitted light corresponding to the amount of bound anti-HIV and/or anti-HTLV-I-antibody is quantified by means of a photomultiplier. The fluorescence intensity is evaluated in comparison to the fluorescence intensity of uninfected cells. However, for this kind of detection system it would be preferred to employ a cell line which expresses viral antigens at a higher frequency such as, e.g., cloned and selected variants of the cell lines 191 TJ (ECACC, 86090501) and HUT 78 (ATCC, TIB 161).

The test is carried out as described in EXAMPLE 17. After the last washing step the cells are resuspended in a suitable volume of PBS and transferred into cuvettes for measurement.

EXAMPLE 19

Immunofluorescence test for the simultaneous determination of anti-HIV antibodies and anti-HTLV-I antibodies in undiluted serum and full blood.

In the same manner as in EXAMPLE 11 the detection of the antibodies may be carried out also with undiluted serum and full blood, respectively, as in EXAMPLES 8 and 9.

In the same manner as in EXAMPLE 10 results may also be obtained within 20 minutes.

EXAMPLE 20

Use of dry blood samples in the immunofluorescence test for anti-HIV-antibodies.

As the whole blood to be investigated there is employed capillary blood collected by puncture. About 10 microliters of blood are placed on a filter paper leaflet (preferably paper grades such as 3MM paper, by Whatman, or No. MN 440 or MN 827, by Macherey & Nagel) having a diameter of 5 mm and allowed to dry thereon. These dried blood samples may be stored at room temperature over a period of from several days to weeks.

It is preferred to use a plastic foil having holes 3 mm in diameter in the slide mask format, filters (for example, 3MM filters by the firm Whatman, 5 mm in diameter, or MN 440 or MN 827 filters, by the firm Macherey & Nagel, 5 mm in diameter) have been glued on the seams of the holes. However, filters made of stiff paper, and preferably those having slide mask format, may also be used, wherein either the entire mask has been plasticized or been rendered hydrophobic, or the filter disklet mark has been applied with hydrophobic print material penetrating the filter (Figures 2 and 3). In a further preferred embodiment plastic strips may be used, which preferably are made of a hydrophobic material, onto which circular or angular filter paper leaflets have been glued. The grades mentioned above are preferred for use as the filter papers. Narrow test strips as conventionally used in medical diagnostics have proven to be particularly suitable.

It is preferred to glue the angular, and preferably square or round, paper filters spaced apart at the same distances as the slide hole positions onto a plastics strip of 6 mm width. If on the slide a further test series, for example with HIV-infected cells, is to be installed, then three filters spaced apart at hole distances are attached to the strip. For the analysis the test strip is positioned over the hole positions so that up to 6 strips may be eluted over the densely grown cell population. The projecting ends of the strips are available for purposes of correct positioning and of coding.

Prior to the elution of the antibody the filter is preferably reconstituted in a moist chamber at 37 °C with 30 microliters of PBS/1% of BSA for at least two hours. In the course thereof the filter paper must not rest on an absorptive substrate, but has to be supported by, for example, glass or Parafilm, to prevent the materials of the individual positions from being mixed. Ten microliters of PBS/1% of BSA are placed onto the application areas. Then each filter is placed directly on an application area and provided with a layer of 20 microliters of PBS/BSA.

The elution/bonding of the antibody is effected in a moist chamber at 37 °C during 30 minutes. Then the slide is rinsed, preferably with PBS, and washed in PBS tempered at 37 °C for 5 minutes. The slides are briefly rinsed with twice distilled water and then allowed to dry. All of the application areas on the dry slide are coated with 10 microliters of FITC-labelled secondary antibody. The slides are incubated in a moist chamber for 30 minutes and then washed as in EXAMPLE 7. The test evaluation is done in the conventional manner.

EXAMPLE 21

Detection of antibodies against HIV from mortfied tissue.

Serum residues from mortified tissue may be investigated for antibodies against HIV by applying about 10 to 15 microliters onto a filter as described in EXAMPLE 19, allowing the sample to dry and working up as is done with dried blood.

EXAMPLE 22

Detection of antibodies from dried blood traces.

Dried blood material secured for criminological or forensic purposes may also be investigated for the presence of anti-HIV antibodies: The dried blood sample is placed on a filter paper leaflet and processed according to the above-described method (EXAMPLE I9). Preferably the filter platelet should consist of a lighter paper grade such as, for example, Whatman 54I paper.

## EXAMPLE 23

Detection of antibodies from sputum and sperm.

In many cases of HIV-infected patients antibodies will be detectable also in sputum or sperm. The tests disclosed here are suitable also for these investigations. For this purpose, said secretions are applied instead of whole blood onto a filter as described in EXAMPLE I9 and subjected to further work-up after drying.

## Claims

I. A method for the detection of antibodies against human and animal retroviruses, and more specifically T-lymphotropic viruses, by allowing a sample to be investigated to react with a human or animal cell line infected with human or animal retroviruses, and more specifically T-lymphotropic viruses, and subsequently labelling by means of a second antibody directed against human antibodies or antibody fragments bearing a marker, characterized in that a cell line is used only part of the cells of which after infection with human and animal retroviruses, and more specifically T-lymphotropic viruses, detectably expresses viral antigens and wherein the negatively-reacting cells are used as internal standard.

2. The method according to claim I, characterized in that the antibodies to be detected are antibodies against retroviruses causatively correlating with the diseases AIDS, ARC (AIDS Related Complex) or lymphadenopathy.

3. The method according to claims I and 2, characterized in that the antibodies to be detected are antibodies against HIV viruses, HTLV-I viruses and/or STLV-I viruses.

4. The method according to claims I to 3, characterized in that the antibodies to be detected are antibodies against variants of the HIV virus.

5. The method according to claim 4, characterized in that the variants of the HIV virus are LAV-2 or HTLV-IV viruses.

6. The method according to claims I to 5, characterized in that antibodies against HTLV-I viruses are detected simultaneously besides antibodies against HIV viruses and antibodies against variants of HIV viruses.

7. The method according to claims I to 6, characterized in that a cell line transformed by HTLV-I viruses or STLV-I viruses is used which constitutively expresses HTLV-I antigen or STLV-I antigen in the external cell membrane in an amount of more than 50%.

8. The method according to claims I to 7, characterized in that the cell line constitutively expresses STLV-I antigens which to a high degree cross-react with anti-HTLV-I antibodies.

9. The method according to claims I to 8, characterized in that the cell line is capable of forming syncytia.

I0. The method according to claims I to 9, characterized in that the described cells are HUT I02 cells (ATCC, TIB I62) or I9I TJ cells (ECACC 86090501).

II. The method according to claims I to 5, characterized in that the cell line HUT 78 (ATCC, TIB I6I) is employed.

I2. The method according to claims I to II, characterized in that fixed cells are employed.

I3. The method according to claims I to II, characterized in that cells are employed which have been in situ and natively fixed with aldehydes and and are used as a non-infectious cellular reagent for the antibody detection.

I4. The method according to claims I to I3, characterized in that the second antibodies employed against antigen-antibody complexes have bound at least one moiety which either absorbs electromagnetic waves, is fluorescent, chemiluminescent, bioluminescent, radioactive, or is labelled with an enzyme.

I5. The method according to claims I to I4, characterized in that a cellular ELISA, a cellular RIA or the cellular immunofluorescence microscopy is employed for detecting the antibodies against human and animal retroviruses, and more specifically T-lymphotropic viruses.

I6. The method according to claims I to I4, Characterized in that the cytofluorimetry, the immunofluorescence spectroscopy, EIA (Enzyme Immuno Assay) or an ELISA is employed for detecting the antibodies against human and animal retroviruses, and more specifically T-lymphotropic viruses.

I7. The method according to claims I to I6, characterized in that the detection of antibodies against human and animal retroviruses is carried out using a sample originating ·from dry blood traces, undiluted serum, whole blood, mortified tissue, sputum, saliva and/or sperm.

I8. The method according to claim I7, characterized in that the whole blood is dried on filter paper and is reconstituted prior to the evaluation.

19. Use of a cell line infected with human or animal retroviruses, and more specifically T-lymphotropic viruses, for the detection of antibodies against human or animal retroviruses, and more specifically T-lymphotropic viruses, wherein only partially viral antigens of the infecting human or animal retrovirus, and more specifically T-lymphotropic virus, are expressed and wherein the negatively-reacting cells are used as internal standard.

20. Use of a cell line according to claim 19, characterized in that it has been transformed by HTLV-I viruses or STLV-I viruses and constitutively expresses HTLV-I antigen or STLV-I antigen in the external cell membrane in an amount of more than 50%.

21. Use of a cell line according to claim 19 for the simultaneous detection of antibodies against various human and animal retroviruses, and more specifically T-lymphotropic viruses.

22. Use of a cell line according to claims 19 to 21, characterized in that the cell line is capable of forming syncytia.

23. Use of a cell line according to claims 19 to 22, characterized in that the cell line(s) HUT 78 (ATCC, TIB 161), 191 TJ (ECACC, 860905OI) and/or HUT 102 (ATCC, TIB 162) is/are employed.

24. Agent for the detection of antibodies against human and animal retroviruses, and more specifically T-lymphotropic viruses, containing fixed cells of a cell line infected with human or animal retroviruses, and more specifically T-lymphotropic viruses and only in part detectably expressing viral antigens, fixed cells of a cell line transformed by human or animal retroviruses, and more specifically T-lymphotropic viruses, whereby the cell line constitutively expresses antigen of human and animal retroviruses, and more specifically T-lymphotropic viruses, and secondary antibody directed against human antibodies and bearing a marker.

25. A device for carrying out the method according to claims 1 to 18, consisting of a laminate comprising

a) an upper hydrophobic film including recesses;

b) a lower hydrophobic film including recesses coaxial to the recesses in the upper hydrophobic film;

c) an absorptive material positioned therebetween which on all sides projects beyond the edges of the recesses of the films; and

d) optionally an upper and/or lower removable cover film applied over the upper and/or lower hydrophobic film.

26. A device for carrying out the method according to claims 1 to 18, consisting of a hydrophobic film including recesses glued over with an absorptive material and optionally a removable cover film attached to the upper and/or lower surface of the hydrophobic film.

27. A device for carrying out the method according to claims 1 to 18, consisting of a strip-shaped hydrophobic film onto which pieces of an absorptive material have been applied in spaced relations.

28. A device for carrying out the method according to claims 1 to 18, characterized in that the hydrophobic film consists of a paper strip rendered hydrophobic except for recesses, or the recess has been separated from its environment by a hydrophobic seam.

29. The device according to claims 25 to 28, characterized in that it is in the form of long strips capable of being wound up.

FIG.1a

FIG.1b

FIG. 2

FIG. 3